**Europäisches Patentamt**

**European Patent Office**

**Office européen des brevets**

(19)

(11) Veröffentlichungsnummer: **0 386 766**

**A1**

(12) # EUROPÄISCHE PATENTANMELDUNG

(21) Anmeldenummer: 90104455.2

(22) Anmeldetag: 08.03.90

(51) Int. Cl.5: **C12N 15/00, C12N 15/85**

(30) Priorität: 09.03.89 DE 3907679

(43) Veröffentlichungstag der Anmeldung:
**12.09.90 Patentblatt 90/37**

(84) Benannte Vertragsstaaten:
**AT BE CH DE DK ES FR GB GR IT LI LU NL SE**

(71) Anmelder: **Max-Planck-Gesellschaft zur Förderung der Wissenschaften e.V.
Bunsenstrasse 10
D-3400 Göttingen(DE)**

(72) Erfinder: **Gruss, Peter, Prof.
Hainbundstrasse 9
D-3400 Göttingen(DE)**
Erfinder: **Zimmer, Andreas, Dr., Nat.Inst.of Mental Health
Lab.of Cell Biology, Building 36/room 3A-17
Bethesda, MD 20892(US)**

(74) Vertreter: **Huber, Bernhard, Dipl.-Chem. et al
Patentanwälte Dipl.-Ing.H. Weickmann,
Dipl.-Phys.Dr.K. Fincke, Dipl.-Ing.F.A.
Weickmann, Dipl.-Chem.B. Huber, Dr.-Ing.H.
Liska, Dipl.-Phys.Dr.J.Prechtel, Möhlstrasse
22
D-8000 München 80(DE)**

(54) **Verfahren zur gezielten Veränderung eines Gens.**

(57) Zur gezielten Veränderung eines Gens innerhalb des Genoms intakter Säugerzellen durch homologe Rekombination, bringt man eine DNA-Sequenz, welche homolog zu dem zu verändernden Gen ist, jedoch mindestens in einem Nukleotid durch Mutation, Deletion oder Insertion von dessen Sequenz abweicht, durch Mikroinjektion in entsprechende Zellen ein und gewinnt Zellen, in denen die erfolgte Veränderung nachgewiesen werden kann.

EP 0 386 766 A1

Xerox Copy Centre

## Verfahren zur gezielten Veränderung eines Gens

Die Erfindung betrifft ein Verfahren zur gezielten Veränderung eines Gens innerhalb des Genoms intakter Säugerzellen durch homologe Rekombination.

Die Erforschung der Ursachen verschiedener genetisch bedingter Krankheiten und die Lokalisierung der die Krankheit auslösenden Defekte auf molekularer Ebene schreitet dank der in ihren Anwendungen immer präziser und sicherer werdenden Gentechnologie immer schneller voran. Dennoch blieb die Funktion vieler Gene von Interesse vor allem in Säugerzellen bisher noch ungeklärt, unter anderem weil noch keine befriedigende Methodik zur Mutagenese intakter Säugerzellen existiert. Eine ebensolche Methodik würde darüberhinaus zur Korrektur bereits bekannter Gendefekte benötigt.

Bisher wird hierzu in ersten Ansätzen in Säugerzellen bei Vorliegen eines defekten Gens versucht, dessen Funktion durch Insertieren eines entsprechenden funktionierenden Gens an anderer Stelle zu ersetzen, es werden dabei jedoch möglicherweise langfristig wirksame Veränderungen an anderer Stelle als der zu mutierenden hervorgerufen, weshalb diese Methode für eine eventuelle Krankheitsbehandlung an Säugerzellen ausscheidet.

Ein anderer wesentlicher Nachteil bei bisherigen experimentellen Verfahren in Säugerzellen zur Mutagenese über homologe Rekombination liegt darin, daß zur Detektion der erfolgten Mutation das Einbringen eines Markergens notwendig ist. Auch bei diesen Markergenen, die an dieser Stelle bzw. überhaupt im Genom Fremdgene sind, muß mit großer Wahrscheinlichkeit mit unerwünschten Nebenwirkungen gerechnet werden.

Auch ist die Erfolgsquote bei den bisher verwendeten Verfahren zur in vivo-Mutagenese, auch "gene targetting" genannt, an Säugerzellen noch relativ gering.

Es ist daher eines der großen Ziele der Weiterentwicklung der Gentechnologie und auch die Aufgabe der vorliegenden Erfindung, ein Verfahren bereitzustellen, das die unmittelbare Veränderung eines spezifischen Teils des Säugerzellgenoms in lebenden Zellen erlaubt, ohne gleichzeitig die Gefahr in sich zu bergen, unbeabsichtigt weitere Mutationen zu bewirken oder zwangsläufig Fremdsequenzen mit einzubringen.

Gelöst wird diese Aufgabe erfindungsgemäß durch ein Verfahren zur gezielten Veränderung eines Gens innerhalb des Genoms intakter Säugerzellen durch homologe Rekombination, bei dem man eine DNA-Sequenz, welche homolog zu dem zu verändernden Gen ist, jedoch mindestens in einem Nukleotid durch Mutation, Deletion oder Insertion von der Sequenz dieses Gens abweicht, durch Mikroinjektion in entsprechende Zellen einbringt und diejenigen Zellen gewinnt, in denen die erfolgte Veränderung nachgewiesen werden kann.

Das erfindungsgemäße Verfahren führt überraschenderweise zu einem besonders hohen Anteil an homologen Rekombinationen an der gewünschten Stelle, wobei in mehr als einer pro 640 Zellen eine homologe Rekombination stattfand. Sogar eine Rekombinationsrate von 1 pro 150 mikroinjizierte Zellen konnte bereits mit dem erfindungsgemäßen Verfahren erreicht werden. Dies ist gegenüber dem Verhältnis von homologer zu illegitimer Rekombination nach bisher in Säugerzellen angewendeten Verfahren eine außerordentliche Erhöhung der Effizienz. Weiter kann im erfindungsgemäßen Verfahren die erfolgte Veränderung nachgewiesen werden, ohne daß die Einführung eines Markergens und eine entsprechende Selektion über dieses Markergen stattfinden muß. Die Veränderung kann nach Klonieren der mikroinjizierten Zellen durch Southern Blotting oder auch Sequenzierung nachgewiesen werden.

In einer besonders bevorzugten Ausführungsform der Erfindung wird der Nachweis der erfolgten Veränderung anhand der PCR(polymerase chain reaction)-Methodik (R.K. Saiki et al., Science 239, (1988) 487-491) geführt. Hierbei kann ohne vorheriges Klonieren der Zellen bereits festgestellt werden, ob die gewünschte Mutation stattgefunden hat. Die Methode ist hierbei so sensitiv, daß bereits wenige DNA-Moleküle zuverlässig analysiert werden können, ohne daß eine große Anzahl von Zellen benötigt wird. Die Methode kann dabei auch in einer Weise angewandt werden, daß sogar Punktmutationen nachweisbar sind.

Zum Nachweis werden 2 Primer verwendet, von denen einer komplementär zu der sich vom Gen unterscheidenden Stelle der DNA-Sequenz ist. Nur bei erfolgter Rekombination kann von diesem Primer aus eine Amplifikation erfolgen und nachgewiesen werden.

Als DNA-Sequenz, welche homolog zu dem zu verändernden Gen ist, jedoch mindestens an einer Stelle von dessen Sequenz abweicht, kann jede natürlich isolierte und gegebenenfalls klonierte, aber auch synthetisch hergestellte DNA-Sequenz verwendet werden. Hierbei geht man bisher davon aus, daß die Länge der Homologie direkt proportional zur Häufigkeit des Genkonversions-Ereignisses ist, wogegen die Länge der Heterologie dazu umgekehrt proportional ist. Es ist daher besonders bevorzugt, eine DNA-Sequenz zu verwenden, welche nur an den nötigen Stellen von der zu verändernden genomischen Sequenz abweicht, jedoch eine möglichst große Homologie aufweist, woraus sich ergibt, daß je länger die verwendete DNA-Sequenz ist, desto höher die Rekombinationsrate ist.

In einer bevorzugten Ausführungsform der Erfindung werden als Säugerzellen somatische Stammzellen verwendet. Bisherige Erkenntnisse über solche Stammzellen und Gentransfer in solche Zellen sind beispielsweise bei P.M. Dexter und D. Boettinger, Gene Transfer into Hemapoetic Cells by Retroviral Vectors, ISI, Atlas of Science, 167-174, zusammengefaßt. Die erfindungsgemäße Veränderung des Genoms von somatischen Stammzellen ermöglicht nach Einbringen der Zellen in ein Säugetier, einen vorhandenen und bekannten Defekt zu beheben, da die Stammzellen zu funktionellen Zellen differenzieren und defekte Populationen im Körper sukzessive ersetzt werden. Als Anwendungsbeispiel für die Korrektur von defekten Genen durch (Wieder-)Einbringen von erfindungsgemäß mutierten Stammzellen können Blutdefekte, wie Faktor VIII-Defizienz (Bluterkrankheit) genannt werden. Eine weitere Anwendungsmöglichkeit ergäbe sich, wenn in Stammzellen, welche zu Langerhans'schen Inselzellen differenzieren, beschriebene Defekte im Insulin-Gen "korrigiert werden könnten", so daß hier die Chance besteht, gegen die Zuckerkrankheit auf Gen-Ebene anzugehen. Somatische Zellen werden in Kultur entweder auf einer sogenannten "Feeder-cell"-Schicht gehalten, d.h. einer Schicht mitotisch inaktiver, nicht mehr teilungsfähiger Zellen, oder aber mit Hilfe von Wachstumsfaktoren (DIA: A.G. Smith et al., Nature, Vol. 336, (1988), Seite 688, LIF: R.L. Williams et al., Nature 336 (1988), Seite 684) an der Differenzierung gehindert und dadurch eine Kultivierung ermöglicht.

Für die Landwirtschaft, vor allem die Nutztierzucht, erscheinen Anwendungen des erfindungsgemäßen Verfahrens interessant, welche z.B. auf eine vermehrte Sekretion von Wachstumsfaktoren in den Tieren abzielen, wobei bei der Tierzucht dann das mutierte Gen in die Keimbahn eingebracht werden könnte, etwa durch Zygoteninjektion in ein befruchtetes Ei oder Verwendung von embryonalen Stammzellen.

Eine weitere wichtige Anwendungsmöglichkeit des erfindungsgemäßen Verfahrens unter Verwendung von somatischen oder embryonalen Stammzellen und gegebenenfalls der Zygoteninjektion in ein befruchtetes Ei, liegt in der sich dadurch eröffnenden Möglichkeit, die Funktion bisher unbekannter Gene am Tiermodell untersuchen zu können. So können beispielsweise Stammzellen von Mäusen, welche ein an bestimmter Stelle mutiertes Gen enthalten, in ein befruchtetes Ei injiziert werden, und die Auswirkungen auf ein derart erhaltenes chimäres Tier untersucht werden. Eine weitere außerordentlich wichtige Anwendungsmöglichkeit besteht in der Einbringung erfindungsgemäß mutierter Zellen in die Keimbahn von Tieren wie z.B. Mäusen zur Erzeugung eines Mäuse-Modells für an Menschen festgestellte Krankheiten. Hierdurch ergäbe sich die Möglichkeit, die Wirkung von Arzneimitteln am Mäuse-Modell zu untersuchen, wobei dieses Mäuse-Modell eine große Aussagekraft für die Anwendung am Menschen hätte.

Für die Zukunft erscheint das erfindungsgemäße Verfahren auch eine Möglichkeit zu bieten, verbesserte oder veränderte, z.B. resistente, Pflanzen züchten zu können. Hierbei kann prinzipiell dieselbe Methode wie bei Säugerzellen angewendet werden, sobald eine Möglichkeit geschaffen ist, in solche Pflanzenzellen durch Mikroinjektion DNA effizient einzubringen und eine homologe Rekombination zu bewirken.

Zusammenfassend wird es durch das erfindungsgemäße Verfahren in bis heute unerwarteter- und überraschenderweise ermöglicht, in den lebenden Zellen am Zielort und nicht an einer homologen Stelle, eine Veränderung, bei einer somatischen Gentherapie eine Korrektur, durchzuführen, wobei keinerlei zusätzliche Fremd-DNA-Sequenzen in die Zelle eingebracht werden und keine langfristigen Defekte zu befürchten sind.

Die folgenden Beispiele erläutern die Erfindung in Verbindung mit den Abbildungen weiter.

Fig. 1 zeigt schematisch die Modifizierung des Hox 1.1-Gens;

Fig. 2 zeigt eine Southern-Analyse der PCR-anplifizierten genomischen DNA aus mikroinjizierten 3T3- (a) und D3-Zellpools (b) und schematisch die PCR-Strategie (c);

Fig. 3 zeigt eine Southern-Analyse von KpnI-verdauter genomischer DNA aus klonierten embryonalen Stammzellen (a, b, c) und schematisch das mutierte und das normale Hox 1.1-Allel sowie die erwarteten Restriktionsfragmente; und

Fig. 4 zeigt einen Southern-Blot der PCR-amplifizierten DNA aus Schwanz-Biopsien von zwei Würfen genetisch veränderter Mäuse.

**Beispiel 1**

Mutagenese des Hox 1.1-Gens.

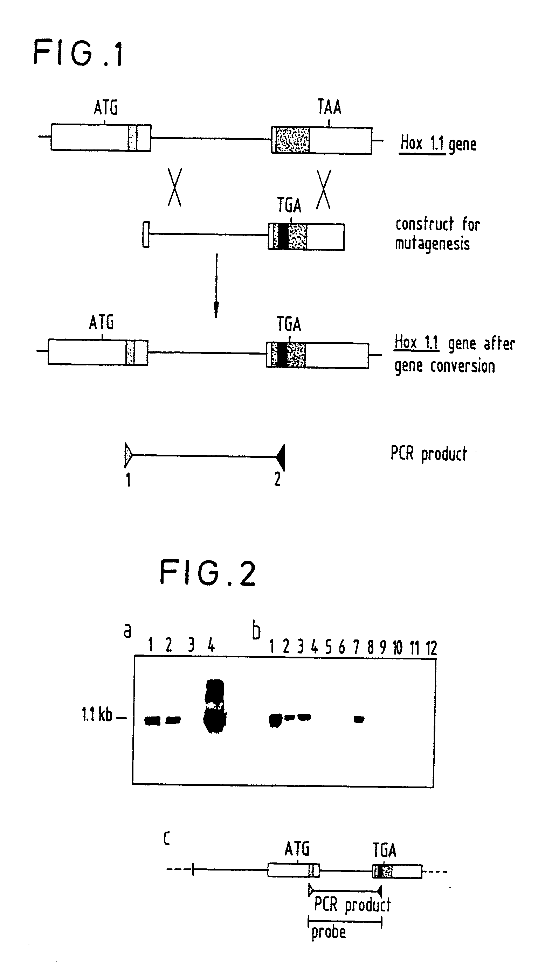

Ein Fsp1-Fragment des Hox 1.1-Gens (Nukleotide 367 bis 1937, gezählt ab dem Startkodon (siehe Fig. 1) (A.M. Colberg-Poley et al., Nature 314 (1985), 713-717, M. Kessel et al., Proc. Natl. Acad. Sci. USA 84 (1987) 5306-5310) wurde in Bluescript (Stratagene) kloniert und ein 20 bp Oligonukleotid in die EcoRI-Stelle der Homöobox insertiert. Die Sequenz des insertierten Oligonukleotids war AAT TGT GAG GTA CCG CTG AC. Dieses in Fig. 1, Zeile 2 dargestellte Konstrukt wurde durch Mikroinjektion in den Kern von NIH-3T3-Zellen (200 bis 600 Zellen) oder D3-Zellen (T.C. Doetschmann et al., J. Embryol. exp. Morph. 87 - (1985) 27-45) (50 bis 200 Zellen) bei einer Konzentration von 300 ng/ml, entsprechend 5 Molekülen DNA pro Zelle, mit einem Injektionsvolumen von $10^{-11}$ ml mikroinjiziert. Die Zellen wurden 4 bis 7 Tage lang nach der Mikroinjektion wachsen gelassen, trypsiniert und auf dieselbe Platte erneut ausgebracht. Die Zellen wurden wieder nach 4 bis 7 Tagen gesammelt. Die Hälfte der Zellen wurde zur DNA-Analyse verwendet, der Rest in flüssigem Stickstoff aufgehoben. Die genomische DNA, bzw. ein Kontrollplasmid (1 μg bzw. 15 pg) wurden 30 PCR-Zyklen unter Verwendung von hitzestabiler Taq-Polymerase (Saiki, R.K. et al., Science 239, 487-491 (1988), Letson, A. und Liskay, R.M., Genetics, 117, 1987, 759-769) in einer 50 μl Reaktionsmischung, enthaltend 10 % Dimethylsulfoxid, 67 mM Tris-HCl (pH 8,8), 16,6 mM $NH_4SO_4$, 6,7 mM $MgCl_2$, 10 mM 2-Mercaptoethanol, 170 μg/ml Rinderserumalbumin (BSA), 450 μM jedes der vier Deoxyribonukleosidtriphosphate (dATP, dCTP, dTTP, dGTP) und 0,5 μM zweier Primer unterworfen. Der erste Primer war ein synthetisches Oligonukleotid der Sequenz TTC CGC ATC TCA CCC TGG AT, der spezifisch ist für eine Sequenz im ersten Exon des Hox 1.1-Gens und auf der 5'-Seite der Fsp1-Schnittstelle bindet, und der zweite Primer eine zu dem bereits obengenannten synthetischen Oligonukleotid identische DNA-Sequenz. Die Proben wurden mit Paraffin überschichtet und in jedem Zyklus eine Minute lang auf 91,5°C erhitzt, um die DNA zu denaturieren, eine Minute lang auf 55°C abgekühlt, um die Primer sich anheften zu lassen und auf 70°C 6 Minuten lang erhitzt, um die Polymerase zu aktivieren. Da das normale Hox 1.1-Allel keine Bindungsstelle für den zweiten Primer enthält und das mikroinjizierte Fragment keine Bindungsstelle für den ersten Primer enthält, konnten nur in erfindungsgemäß mutierten Zellen durch Polymerase-Kettenreaktion das gesuchte Fragment amplifiziert werden. Aliquote der Reaktionsmischung (10 μl) wurden in einem 1%igen Agarosegel elektrophoretisiert, auf Gene Screen Plus (DuPont) transferiert und in 50 % Formamid, 1 M NaCl und 1 % SDS mit einer Hybridisierungssonde untersucht, die mit $^{32}$P auf eine Aktivität von $1\times10^9$ cpm/μg unter Verwendung eines Multiprime-Kits (Amersham) markiert war und mit $5\times10^5$ cpm/ml verwendet wurde. Die Filter wurden in 2xSSC, dann in 2xSSC-1 % SDS und schließlich in 0,1xSSC (1xSSC: 8,765g/l NaCl, 4,41 g/l Natriumcitrat, pH 7,0) bei 65°C gewaschen. Fig. 2 zeigt ein Autoradiogramm der getrockneten Filter, Teil a nach 2 Stunden und Teil b nach 5 Stunden Exposition. Hierbei zeigt Fig. 2a eine Southern-Analyse von PCR-amplifizierter genomischer DNA aus mikroinjizierten 3T3-Pools (Spuren 1 bis 3) und des Plasmids pH 1.1/O-BF in Spur 4 als Kontrolle. Dieses Plasmid enthält das gesamte Hox 1.1-Gen und das insertierte Oligonukleotid. In Teil b der Abbildung ist eine Southern-Analyse von PCR-amplifizierter genomischer DNA aus mikroinjizierten D3-Zellpools gezeigt. Fig. 2c zeigt schematisch das Plasmid pH1.1/O-BF. Ein 3,643 Basenpaar langes 5-BamHI-FspI- Fragment des Hox1.1-Gens (Nukleotide - 1,711 bis + 1,932, bezogen auf das Translations-Startkodon) wurde in Bluescript (Stratagene) kloniert und das unterbrechende Oligonukleotid in die EcoRI-Stelle der Homöobox kloniert.

**Beispiel 2**

Weitere Untersuchung der Stammzellen

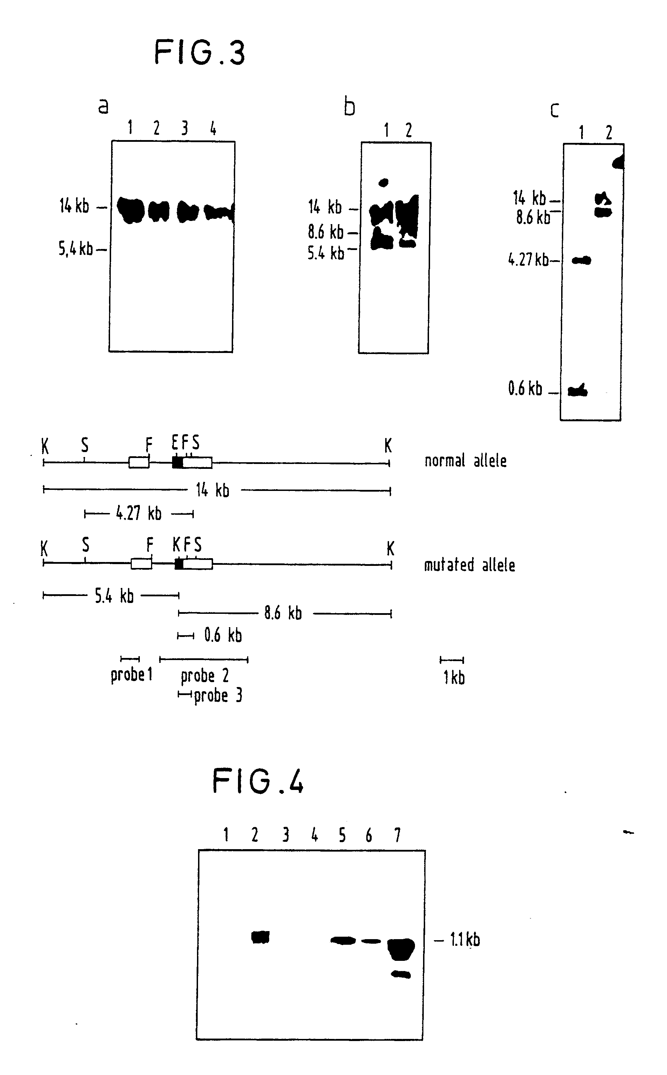

Zur weiteren Analyse wurden zwei Zellpools der embryonalen Stamm(ES)-Zellen D3 aus Beispiel 1 (G.R. Dressler und P. Gruß, Trans Genet. 4, 214-219 (1988), T.C. Doetschmann et al., J. Embryol. Exp. Morph. 87 (1985), 27-45) verwendet. Einzelne Zellklone aus diesen Pools wurden durch Aufnehmen von individuellen Zellen mit Glaskapillaren (M.A. Rudnicki & M.W. McBurney in Teratocarcinomas and Embryonic Stem Cells, (ed. E.J. Robertson) 19-49 (Publisher, Town, 1987) aufgenommen. Von diesen Zellen wurden klonale Zellinien hergestellt durch Plattieren zuerst in Schalen mit 96 Vertiefungen, dann in Schalen mit 24 Vertiefungen mit 1,5 cm Durchmesser und dann in 6 cm Gewebekulturschalen. Aus der Hälfte dieser Zellinien wurde DNA isoliert, die andere Hälfte in flüssigem Stickstoff aufgehoben. Während der gesamten Klonierungsprozedur wurden die ES-Zellen auf sogenannten "Feeder"-Zellen in Kultur gehalten. Aliquots (10 μg) der genomischen DNA wurden mit KpnI verdaut, in 0,8%igen Agarosegelen einer Elektrophorese unterworfen und Gene Screen Plus transferiert. Die Filter wurden mit $^{32}$P markierten Sonden, wie im Beispiel 1 beschrieben, hybridisiert und ebenso wie dort beschrieben, gewaschen. Da das insertierte Oligonukleotid eine KpnI-Restriktionsstelle besitzt, wurde das Auftreten einer neuen 5,4 kb Bande in homolog rekombinierten Klonen zusätzlich zu der 14 kb Bande des normalen Allels nach Hybridisierung mit einer für das erste Exon spezifischen Sonde (Probe 1) in Fig. 2d erwartet. Das Auftreten dieser Bande ist in der Spur 1 von Fig. 2a gezeigt. Die ES-Zell-DNA war mit Feeder-Zell-DNA kontaminiert worden, da verhindert wurde, daß die ES-Zellen große Kolonien während des subklonierens bildeten. Daher stellt das 14 kb Hox1.1-Fragment nicht nur das Wildtyp-Allel aus ES-Zellen dar, sondern auch Wildtyp-Allele aus den Feeder-Zellen. Insgesamt wurden 161 individuelle Klone aus Pool 1 und 165 Klone aus Pool 7 durch Southern Blotting analysiert. Um die Southern-Analyse zu bestätigen und auszuweiten, wurden Klone aus beiden Pools kultiviert, bis große ES-Zellkolonien sich auf den Feeder-Zellen bildeten. Die relativen Mengen von ES-Zell-DNA war dann sehr viel höher, wie aus Fig. 3b und C zu entnehmen ist. Ein Southern-Blot von Kpnl verdauter DNA aus einer klonalen Linie, die abgeleitet war aus Pool 1, wurde mit Probe 1 (Fig. 3b, Zeile 1) und Probe 2 (Fig. 3b, Spur 2) hybridisiert. In Fig. 3c sind Einzelheiten der Proben dargestellt. Die relativen Intensitäten der 5,4 kb und der 8,6 kb Banden entsprechen den 5'- und 3'-Fragmenten (siehe Fig. 3d) im Vergleich mit der des 14 kb Fragments, wodurch gezeigt wird, daß mindestens 50 % der ES-Zellen das mutierte Allel enthalten. DNA einer klonalen Linie aus Pool 7 wurde mit Stul/Kpnl (Fig. 3c, Spur 1) und Kpnl (Fig. 3c, Spur 2) verdaut und Southern-Blots in der Folge mit Probe 3 (siehe Fig. 3d) hybridisiert. Die entstehenden 600 bp und 4,27 kb Fragmente (Fig. 3c, Spur 1) sind abgeleitet aus den mutierten und normalen Allelen (siehe Fig. 3 für genauere Angaben). Das mutierte und das normale Allel sind in gleichen Mengen in dem Stul/Kpnl und dem Kpnl-Verdauungsansatz (Fig. 3c, Spuren 1 und 2) repräsentiert, wodurch gezeigt wird, daß die ES-Zellen aus Pool 7 klonal sind. Eine Karyotyp-Analyse des Klons, der aus Pool 1 abgeleitet ist, zeigte, daß 70 % der Zellen 40 Chromosomen enthielten. Es wird daher angenommen, daß die geringere Anwesenheit des mutierten Allels in dieser Zellinie zurückzuführen ist auf Kontamination mit nicht mutierten ES-Zellen und nicht auf eine chromosomale Instabilität hindeutet. Es wurde abgeschätzt, daß das Verhältnis von homologer zu illegitimer Rekombination 1:30 ist, da ungefähr 20 % der mikroinjizierten Zellen stabil transformiert wurden nach nuklearer Mikroinjektion (M.R. Capecchi, Cell 22 (1980) 479-488). Die homologe Rekombination fand in 1 pro 150 mikroinjizierten Zellen statt. Dies ist erstaunlich hoch, verglichen mit vergleichbaren Untersuchungen (K.R. Thomas & M.R. Capecchi, Cell 51 (1987) 503-512, O. Smithies et al., Nature 317 (1985) 230-234, K.R. Thomas et al., Cell 44 (1986), 419-428).

**Beispiel 3**

Die in Beispiel 1 erhaltenen embryonalen Stammzell-Linien, die in Fig. 3a und 3b (Wurf 1: Spuren 1 bis 4 der Fig. 4, Wurf 2: Spuren 5 bis 7 der Fig. 4) gezeigt sind, wurden in Blastocysten von C57BL/6 Mäusen eingebracht und zwei Würfe mit einer Gesamtzahl von 4 chimären Tieren erhalten. Um zu bestätigen, daß die chimären Tiere das mutierte Allel enthalten, wurde aus den Schwänzen der Tiere eine Biopsie entnommen und 100 ng von genomischer DNA dieser Biopsien 30 PCR-Zyklen, wie im Beispiel 1 beschrieben, unterworfen. Die Spuren 2, 5, 6 und 7 der Fig. 4 zeigen eine Amplifikation des 1,1 kb Fragments, das das mutierte Allel zeigt (siehe Fig. 1 und 2).

**Ansprüche**

1. Verfahren zur gezielten Veränderung eines Gens innerhalb des Genoms intakter Säugerzellen durch homologe Rekombination, **dadurch gekennzeichnet,** daß man eine DNA-Sequenz, welche homolog zu dem zu verändernden Gen ist, jedoch mindestens in einem Nukleotid durch Mutation, Deletion oder Insertion von dessen Sequenz abweicht, durch Mikroinjektion in entsprechende Zellen einbringt und Zellen gewinnt, in denen die erfolgte Veränderung nachgewiesen werden kann.

2. Verfahren nach Anspruch 1, **dadurch gekennzeichnet,** daß man als Säugerzellen somatische Stammzellen verwendet.

3. Verfahren nach Anspruch 1 oder 2, **dadurch gekennzeichnet,** daß man zum Nachweis der erfolgten Veränderung eine PCR-Reaktion durchführt.

4. Verfahren nach einem der Ansprüche 1 bis 3, **dadurch gekennzeichnet,** daß das zu verändernde Gen ein Gen ist, welches Krankheitserscheinungen hervorruft und man eine DNA-Sequenz verwendet, welche dem gesunden Gen entspricht.

# FIG.1

Hox 1.1 gene

construct for mutagenesis

Hox 1.1 gene after gene conversion

PCR product

# FIG.2

# FIG.3

# FIG.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | NUCLEIC ACIDS RESEARCH, Band 16, Nr. 18, 26. September 1988, IRL Press Ltd, Oxford, GB; H.-S. KIM et al.: "Recombinant fragment assay for gene targetting based on the polymerase chain reaction" * Das ganze Dokument, überhaupt Figur 1 * | 1,2,3,4 | C 12 N 15/00 C 12 N 15/85 |
| P,X | NATURE, Band 338, Nr. 153, 9. März 1989, Seiten 153-156; A.L. JOYNER et al.: "Production of a mutation in mouse En-2 gene by homologous recombination in embryonic stem cells" * Das ganze Dokument * | 1,2,3,4 | |
| P,X | NATURE, Band 338, Nr. 153, 9. März 1989, Seiten 150-153; A. ZIMMER et al.: "Production of chimaeric mice containing embryonic stem (ES) cells carrying a homoeobox Hox 1.1 allele mutated by homologous recombination" * Das ganze Dokument * | 1,2,3,4 | |
| P,X | SCIENCE, Band 244, Nr. 4910, 16. Juni 1989, Seiten 1288-1292, Washington, DC, US; M.R. CAPECCHI: "Altering the genome by homologous recombination" * Seite 1290: "Nonselectable genes" * | 1,2,3,4 | RECHERCHIERTE SACHGEBIETE (Int. Cl.5) C 12 N |
| D,X | CELL, Band 51, 6. November 1987, Seiten 503-512, Cell Press; K.R. THOMAS et al.: "Site-directed mutagenesis by gene targeting in mouse embryo-derived stem cells" * Das ganze Dokument * | 1,2 | |

Der vorliegende Recherchenbericht wurde für alle Patentansprüche erstellt

| Recherchenort | Abschlußdatum der Recherche | Prüfer |
|---|---|---|
| DEN HAAG | 11-06-1990 | CHAMBONNET F.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)